(19) Europäisches Patentamt
European Patent Office
Office européen des brevets

(11) EP 1 404 663 B1

(12) EUROPÄISCHE PATENTSCHRIFT

(45) Veröffentlichungstag und Bekanntmachung des Hinweises auf die Patenterteilung:
**04.05.2005 Patentblatt 2005/18**

(21) Anmeldenummer: **02751052.8**

(22) Anmeldetag: **20.06.2002**

(51) Int Cl.[7]: **C07D 313/12**, C12N 1/14, A61K 31/00, A61P 35/00

(86) Internationale Anmeldenummer:
**PCT/EP2002/006841**

(87) Internationale Veröffentlichungsnummer:
**WO 2003/002549 (09.01.2003 Gazette 2003/02)**

(54) **EUROTINONE UND DERIVATE DAVON, VERFAHREN ZU IHRER HERSTELLUNG UND VERWENDUNG DERSELBEN**

EUROTINONE AND DERIVATIVES THEREOF, METHOD FOR THE PRODUCTION AND USE OF THE SAME

EUROTINONE ET SES DERIVES, PROCEDE PERMETTANT LEUR PREPARATION, ET LEUR UTILISATION

(84) Benannte Vertragsstaaten:
**AT BE CH CY DE DK ES FI FR GB GR IE IT LI LU MC NL PT SE TR**

(30) Priorität: **27.06.2001 DE 10130890**

(43) Veröffentlichungstag der Anmeldung:
**07.04.2004 Patentblatt 2004/15**

(73) Patentinhaber: **Aventis Pharma Deutschland GmbH**
**65929 Frankfurt am Main (DE)**

(72) Erfinder:
- **EDER, Claudia**
  **65719 Hofheim (DE)**
- **KOGLER, Herbert**
  **61479 Glashütten (DE)**
- **TOTI, Luigi**
  **65239 Hochheim (DE)**

(56) Entgegenhaltungen:
**WO-A-01/66783** **GB-A- 2 323 845**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 404 663 B1

Printed by Jouve, 75001 PARIS (FR)

## Beschreibung

Die vorliegende Erfindung betrifft neue Verbindungen der allgemeinen Formel I

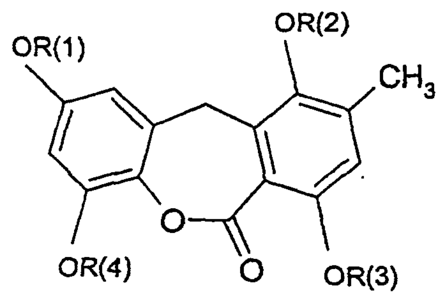

I

worin R(1), R(2), R(3), R(4) unabhängig voneinander Wasserstoff oder einen Alkylrest bedeuten. Die Verbindungen der Formel I sind Inhibitoren der KDR-Kinase und sind aufgrund ihrer antiangiogenetischen Wirkung zur Prävention und/oder Behandlung von malignen Erkrankungen geeignet. Die Verbindungen der Formel I sind erhältlich durch Fermentation des Mikroorganismus Eurotium echinulatum Delacroix (DSM 13872) oder durch chemische Derivatisierung der nach Fermentation des genannten Mikroorganismus erhaltenen Verbindungen. Die Erfindung betrifft somit auch ein Verfahren zur Herstellung der Verbindungen der Formel I, die Verwendung der Verbindungen der Formel I zur Herstellung eines Arzneimittels zur Behandlung von malignen Erkrankungen und von Krankheiten die durch Hemmen der KDR-Kinase behandelt werden können, sowie pharmazeutischen Zubereitungen mit einem Gehalt an mindestens einer Verbindung der Formel I.

Krebs ist eine zumeist tödlich verlaufende Erkrankung von Mensch und Tier, die durch das unkontrollierte Wachsen von körpereigenen Zellen verursacht wird. Krebs ist die Bezeichnung für die Bildung von bösartigen Geschwülsten (Malignome), von Neoplasmen (Tumoren bzw. Carcinoma) oder für die maligne Entartung sowie Reifungsstörung weißer Blutzellen (Leukämie, Blutkrebs). Krebs- oder Tumorzellen entstehen durch Umwandlung körpereigener Zellen. Die Bösartigkeit der Krebszelle drückt sich in der Autonomie des Wachstums aus, das heißt in ihrer Fähigkeit, ungehemmt und ohne Einordnung in den Bauplan der Organe und unter Gewebszerstörung infiltrierend zu wachsen. Ein sicheres Zeichen der Malignität ist die Bildung tumorferner Absiedlungen (Metastasen) nach hämatogener oder lymphogener Ausbreitung von Tumorzellen. Krebs gehört zu den häufigsten Todesursachen des Menschen und deshalb besteht ein großer Bedarf an Methoden und Mitteln zur Heilung oder Behandlung von malignen Entartungen.

Die Möglichkeit einer Therapie maligner Tumoren umfaßt neben der - wenn möglich radikalen - operativen Entfernung des Tumors, die radiologische Therapie mit Röntgenstrahlen, $\alpha$-, $\beta$-, $\gamma$-Strahlen, die Immuntherapie und die Chemotherapie. Die Immuntherapie ist derzeit nur in eingeschränktem Maß anwendbar. Unter der Chemotherapie von Tumoren versteht man die Gabe von Zellgiften (Zytostatika) zur Behandlung von Tumoren und von verbliebenen Tumorzellen nach lokaler chirurgischer Behandlung oder Bestrahlung. Diese Stoffe greifen spezifisch in bestimmte Vorgänge der Zellteilung ein, so daß Gewebe mit hohem Anteil an sich teilenden Zellen, wie das rasch wachsende Tumorgewebe, empfindlicher reagieren. Zum Einsatz kommen alkylierende Verbindungen, wie z. B. das Cyclophosphamid (The Merck Index, 12. Ed. Seite 463), Antimetabolite, wie das Methotrexat (The Merck Index, 12. Ed. Seite 1025), Alkaloide, wie das Vincristin (The Merck Index, 12. Ed. Seite 1704) und Antibiotika, wie das Daunomycin (The Merck Index, 12. Ed. Seite 479) sowie das Adriamycin (The Merck Index, 12. Ed. Seite 581-582). All diese Agenzien haben aber aufgrund von massiven Nebenwirkungen große Nachteile, so daß der Tod der Erkrankten nur hinausgezögert, nicht jedoch abgewendet wird. Zudem treten bei entarteten (Krebs-) Zellen Resistenzen gegen die angewandten Mittel auf, die derzeitigen Medikamente wirken dann nicht mehr zytostatisch, wohl aber toxisch infolge der Nebenwirkungen. Zudem hat sich gezeigt, daß eine kombinierte bzw. sequentielle Anwendung von Zytostatika die Wirksamkeit eines einzelnen Zytostatikums (Monotherapie) übertrifft und dadurch möglich ist, daß sich die erheblichen Nebeneffekte bei der Polychemotherapie nicht addieren. Aus all diesen Gründen werden neue Chemotherapeutika dringend benötigt und deshalb weltweit gesucht.

Das Wachstum von Tumoren setzt eine ausreichende Versorgung des Tumors mit Sauerstoff voraus, die nur durch eine ausreichende Durchblutung (Vaskularisierung) des Tumors gewährleistet ist. Tumore sind nicht zur Bildung neuer Blutgefäße (=Angiogenese) fähig sondern müssen das sie umgebende Bindegewebe zur Angiogenese veranlassen.

Die Bildung neuer Blutgefäße ausgehend von einem schon existierenden Gefäßsystem ist für die embryonale Entwicklung und die Organentwicklung von zentraler Bedeutung. Eine abnormal gesteigerte Angiogenese wird u.a.

bei rheumatoider Arthritis, diabetischer Retinopathie und beim Tumorwachstum beobachtet (Folkman, 1995, Nat. Med., 1, 27-31). Die Angiogenese ist ein komplexer, mehrstufiger Prozeß, der Aktivierung, Migration, Proliferation und Überleben endothelialer Zellen einschließt.

In Verbindung mit anderen Endothel-spezifischen Signalsystemen übertragen die sogenannten vascular endothelial growth factors (VEGFRs) Signale für die endotheliale Zellmigration, Proliferation und das Überleben der Zellen. Die VEGFR-Familie schließt die Subtypen VEGFR-1 (Flt-1), VEGFR-2 (KDR) und VEGFR-3 (Flt-4) ein. Während VEGFR-1 und VEGFR-2 als universelle Regulatoren von endothelialen Zellen fungieren, kontrolliert VEGFR-3 hauptsächlich das Wachstum des lymphatischen Gefäßsystems. VEGFRs spielen eine Schlüsselrolle in sämtlichen Stadien des angiogenetischen Prozesses.

Extensive Studien auf dem Feld der Tumorangiogenese in den letzten 20 Jahren haben eine große Anzahl an potentiellen therapeutischen Targets beschrieben, z.B. Kinasen, Proteasen und Integrine. Dies zog die Entdeckung vieler neuer antiangiogenetischer Agentien nach sich, von denen sich einige bereits in der klinischen Prüfung befinden (Jekunen et al., 1997, Cancer Treatment Rev., 23, 263-286). Angiogenese-Inhibitoren könnten sowohl zur Monotherapie, wie auch in Kombinationstherapie mit anderen Zytostatika im Rahmen einer chemotherapeutischen Tumorbehandlung verwendet werden. Darüber hinaus ist ein Einsatz zur Prävention denkbar um erneutes Wachstum eines Tumors nach erfolgter Therapie zu verhindern.

Die Inhibierung bzw. Regulierung von VEGFR-2 (KDR) ist ein Reaktionsmechanismus, der einen neuartigen Ansatz für die Behandlung einer Vielzahl von soliden Tumoren bietet. So ist die Aktivierung dieses Tyrosinkinase-Rezeptors entscheidend für endotheliales Zellwachstum und Gefäßneubildung bei der Angiogenese und hat somit Einfluß auf das Tumorwachstum und die Bildung von Metastasen. Darüber hinaus gibt es neue Hinweise darauf, daß die Expression von VEGF zum Überleben von Tumorzellen nach einer Strahlen- oder Chemotherapie beiträgt (Lee C: G., Heijn M. et al., 2000, Cancer Research, 60 (19), 5565-70). Dies unterstreicht die Bedeutung einer möglichen synergistischen Wirkung von KDR-Inhibitoren mit den bisher bekannten Zytostatika.

Es ist überraschend gefunden worden, daß der Mikroorganismus Eurotium echinulatum Delacroix (DSM 13872) einen Wirkstoff zu bilden vermag, der eine ausgeprägte inhibierenden Wirkung auf die KDR-Kinase aufweist und somit einen wirksamen Angiogenese-Inhibitor darstellt. Die neue Verbindung wird im folgenden Eurotinon genannt und ist, zusammen mit Eurotinon-Derivaten, Gegenstand der Erfindung.

Die vorliegende Erfindung betrifft daher Verbindungen der allgemeinen Formel I

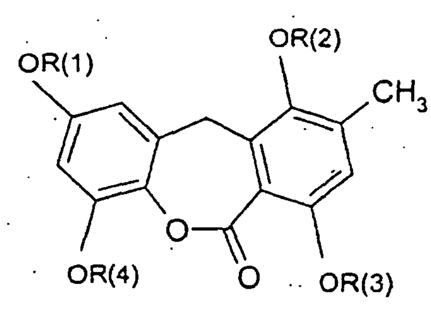

I

worin

R(1), R(2), R(3) und R(4) unabhängig voneinander, jeweils Wasserstoff oder $(C_1\text{-}C_6)$-Alkyl, $(C_2\text{-}C_6)$-Alkenyl, $(C_2\text{-}C_6)$-Alkinyl; $C_3\text{-}C_6$-Cycloalkyl oder $(C_1\text{-}C_3)$-Alkyl-$(C_3\text{-}C_6)$-Cycloalkyl bedeutet, in allen ihren stereochemischen Formen und Gemische dieser Formen in jedem Verhältnis, sowie deren physiologisch verträgliche Salze.

$C_1\text{-}C_6$-Alkyl kann ein geradkettiges oder verzweigtes Alkyl mit 1 bis 6 C-Atomen, wie z.B. Methyl, Ethyl, i-Propyl, tert. Butyl und Hexyl;

$C_2\text{-}C_6$-Alkenyl ein geradkettiges oder verzweigtes Alkenyl mit 2 bis 6 C-Atomen, wie z.B. Allyl, Crotyl und Pentenyl;

$C_2\text{-}C_6$- Alkinyl ein geradkettiges oder verzweigtes Alkinyl mit 2 bis 6 C-Atomen, wie z.B. Propinyl, Butinyl und Pentinyl, bedeuten.

Beispiele für $(C_3\text{-}C_6)$-Cycloalkyl sind Cyclopropyl, Cyclobutyl, Cyclopentyl oder Cyclohexyl.

Die obengenannten Alkylreste können mit einem oder mehreren Resten substituiert sein. Diese können beispielsweise Halogen, wie Chlor, Brom, Fluor; Hydroxy; Alkoxy mit 1-4 C-Atomen, insbesondere Methoxy und/oder Trifluormethyl sein.

Bevorzugt bedeuten R(1) - R(4) in der Formel I jeweils unabhängig voneinander Wasserstoff oder $(C_1\text{-}C_6)$-Alkyl.

Bevorzugt sind insbesonderere Verbindungen der Formel I, in denen

a) R(1)-R(4) Wasserstoff (= Eurotinon);
b) R(1)-R(3) Wasserstoff und R(4) ($C_1$-$C_6$)-Alkyl, insbesonderer Methyl; oder
c) R(1), R(2) Waserstoff und R(3), R(4) ($C_1$-$C_6$)-Alkyl, insbesonderer Methyl

bedeutet; in allen ihren stereochemischen Formen und Gemische dieser Formen in jedem Verhältnis, sowie deren physiologisch verträgliche Salze.

Die Erfindung betrifft somit insbesondere das Eurotinon der Formel

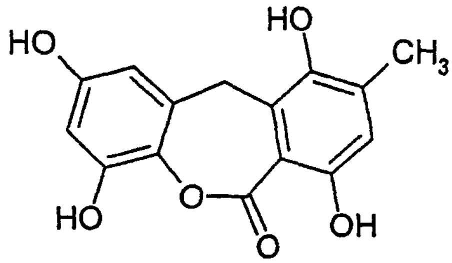

sowie deren physiologisch verträgliche Salze.

Die Erfindung betrifft weiterhin das 2,12-Dimethyleurotinon der Formel

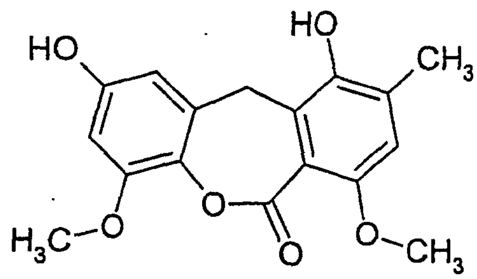

und das 2-Methyleurotinon der Formel

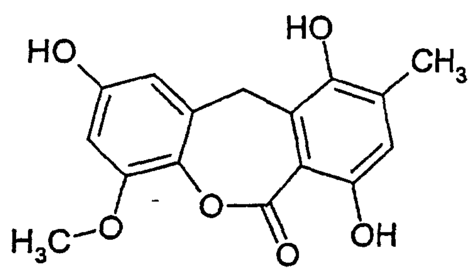

sowie deren physiologiscb verträgliche Salze.

Erfindungsgemäß sind die Verbindungen der Formel I erhältlich durch Fermentation des Mikroorganismus Eurotium echinulatum Delacroix (DSM 13872) oder dessen Varianten oder Mutanten unter geeigneten Bedingungen. Durch anschließende Isolierung der Verbindungen und gegebenenfalls Umwandlung in chemische Derivate, sowie deren physiologisch verträglichen Salze werden die Eurotinone gewonnen.

Die Erfindung betrifft daher weiterhin ein Verfahren zur Herstellung einer Verbindung der Formel I, dadurch gekennzeichnet, daß der Mikroorganismus Eurotium echinulatum Delacroix (DSM 13872) oder dessen Varianten oder Mutanten unter geeigneten Bedingungen in einem Kulturmedium fermentiert wird, bis sich das Eurotinon im Kulturmedium oder im Mikroorganismus anhäuft und anschließend aus dem Kulturmedium oder Mikroorganismus isoliert und

gegebenenfalls in chemische Derivate und /oder physiologisch verträglichen Salze umgewandelt wird.

In der Literatur sind viele Reaktionen zur Alkylierung von Phenolen beschrieben worden. Die Alkylierung der phenolischen OH-Gruppen der vorliegenden Verbindungen kann daher mit an sich bekannten chemischen Reaktionen durchgeführt werden. Eine Derivatisierung zu den alkylierten Derivaten des Eurotinons kann zum Beispiel durch Umsetzung mit Alkylhalogeniden wie Alkylbromiden oder Alkyljodiden, Alkylsulfonsäureestern wie Mesylaten, Tosylaten oder Triflaten sowie Diazoalkanen wie Diazomethylen oder Trimethylsilyl-diazomethan erfolgen.

Im folgenden wird die Erfindung detailliert beschrieben, insbesondere in ihren bevorzugten Ausführungsformen.

Die erfindungsgemäßen Eurotinone werden durch den Pilz Eurotium echinulatum, bevorzugt durch Eurotium echinulatum Delacroix DSM 13872 produziert.

Der Pilz Eurotium besitzt ein gelb/braunes Substratmycel und wenig Luftmycel. Er bildet in Kultur die für Eurötium charakteristischen Fruchtkörper, die Kleistothecien. Die Ascosporen sind abgeflachte sphärische Kugeln. Sie haben einen typischen equatorialen Kamm. Der Pilz ist ubiquitär und bevorzugt trockene Habitate. Als Synonym wird auch noch die Bezeichnung Aspergillus echinulatus gebraucht.

Ein Isolat von Eurotium echinulatum Delacroix, wurde bei der Deutschen Sammlung von Mikroorganismen und Zellkulturen GmbH (DSMZ), Mascheroder Weg 1B, 38124 Braunschweig, Deutschland nach den Regeln des Budapester Vertrages am 15.11.2000 unter der folgenden Nummer hinterlegt: DSM 13872.

Auf einem festen oder flüssigen Kulturmedium, das eine Kohlenstoffquelle und eine Stickstoffquelle sowie die üblichen anorganischen Salze enthält, produziert Eurotium echinulatum Delacroix DSM 13872 das erfindungsgemäße Eurotinon.

Anstelle des Stammes Eurotium echinulatum Delacroix DSM 13872 können auch dessen Mutanten und Varianten eingesetzt werden, die ebenfalls die erfindungsgemäßen Eurotinone synthetisieren.

Solche Mutanten können in an sich bekannter Weise durch physikalische Mittel, beispielsweise Bestrahlung, wie mit Ultraviolett- oder Röntgenstrahlen, oder chemische Mutagene, wie beispielsweise Ethylmethansulfonat (EMS), 2-Hydroxy-4-methoxy-benzophenon (MOB) oder N-Methyl-N'-nitro-N-nitrosoguanidin (MNNG) oder unter Anwendung gentechnischer Methoden erzeugt werden.

Die im folgenden beschriebenen Fermentationsbedingungen gelten für Eurotium echinulatum, das hinterlegte Isolat Eurotium echinulatum Delacroix DSM 13872 sowie Mutanten und Varianten von diesen.

Das erfindungsgemäße Verfahren kann für die Fermentation im Labormaßstab (Milliliter- bis Literbereich) und im industriellen Maßstab (Kubikmetermaßstab) eingesetzt werden. Alle Prozentangaben beziehen sich, wenn nichts anderes angegeben ist, auf das Gewicht. Mischungsverhältnisse bei Flüssigkeiten beziehen sich auf das Volumen, wenn keine anderen Angaben gemacht werden.

Das erfindungsgemäße Verfahren umfasst die Kultivierung von Eurotium echinulatum Delacroix, DSM 13872, seiner Mutanten und/oder Varianten unter aeroben Bedingungen in einem eine Kohlenstoff- und Stickstoffquelle, anorganischen Salze und gegebenenfalls Spurenelemente enthaltenden Kulturmedium. Der Stamm Eurotium echinulatum Delacroix, DSM 13872 bildet auf Glukose-, Stärke-Haferflocken- oder Glycerinhaltigen Nährlösungen das Eurotinon.

Als bevorzugte Kohlenstoffquellen für die Fermentation eignen sich assimilierbare Kohlenhydrate und Zuckeralkohole, wie Glukose, Laktose, Saccharose oder D-Mannit sowie kohlenhydrathaltige Naturprodukte, wie z.B. Malzextrakt oder Hefextrakt. Als stickstoffhaltige Nährstoffe kommen in Betracht: Aminosäuren, Peptide und Proteine sowie deren Abbauprodukte, wie Casein, Peptone oder Tryptone, ferner Fleischextrakte, Hefeextrakte, gemahlene Samen, beispielsweise von Mais, Weizen, Bohnen, Soja oder der Baumwollpflanze, Destillationsrückstände der Alkoholherstellung, Fleischmehle oder Hefeextrakte, aber auch Ammoniumsalze und Nitrate, insbesondere aber auch synthetisch bzw. biosynthetisch gewonnene Peptide. An anorganischen Salzen kann die Nährlösung beispielsweise Chloride, Carbonate, Sulfate oder Phosphate der Alkali- oder Erdalkalimetalle, Eisen, Zink, Kobalt und Mangan enthalten.

Die Bildung des erfindungsgemäßen Eurotinon verläuft besonders gut z.B. in einer Nährlösung, die etwa 0,05 bis 5%, bevorzugt 1 bis 2% Malzextrakt, die etwa 0,05 bis 3%, bevorzugt 0.05 bis 1% Hefeextrakt und 0,2 bis 5%, bevorzugt 0,5 bis 2% Glucose, 0,5 bis 3%, bevorzugt 1.5 bis 3% Haferflocken enthält. Die Angaben in Prozent sind jeweils bezogen auf das Gewicht der gesamten Nährlösung.

Die Kultivierung des Mikroorganismus erfolgt aerob, also beispielsweise submers unter Schütteln oder Rühren in Schüttelkolben oder Fermentern oder auf Festmedium, gegebenenfalls unter Einführen von Luft oder Sauerstoff. Sie kann in einem Temperaturbereich von etwa 15 bis 35°C, vorzugsweise bei etwa 20 bis 35°C, insbesondere bei 25 bis 30°C durchgeführt werden. Der pH-Bereich sollte zwischen 3 und 10 liegen, vorzugsweise zwischen 6.5 und 7.5. Man kultiviert den Mikroorganismus unter diesen Bedingungen im allgemeinen über einen Zeitraum von 48 bis 720 Stunden, vorzugsweise 72 bis 720 Stunden. Vorteilhaft kultiviert man in mehreren Stufen, d. h. man stellt zunächst eine oder mehrere Vorkulturen in einem flüssigen Nährmedium her, die dann in das eigentliche Produktionsmedium, die Hauptkultur, beispielsweise im Volumenverhältnis 1:10-1:100, überimpft werden. Die Vorkultur erhält man z. B., indem man das Mycel in eine Nährlösung überimpft und etwa 20 bis 120 Stunden, bevorzugt 48 bis 72 Stunden,

wachsen läßt. Das Mycel kann beispielsweise erhalten werden, indem man den Stamm etwa 1 bis 40 Tage, bevorzugt 15 bis 20 Tage, auf einem festen oder flüssigen Nährboden, beispielsweise Hefe-Malz-Agar, Haferflocken-Agar oder Kartoffeldextrose-Agar, wachsen läßt.

Der Pilz Eurotium echinulatum Delacroix, DSM 13872 kann die Verbindung Eurotinon in einer Oberflächen- oder Standkultur auf festen Nährböden bilden. Feste Nährböden werden durch Zugabe zum Beispiel von Agar oder Gelatine zu wäßrigen Nährmedien hergestellt. Es ist aber auch möglich das Eurotinon durch Fermentation des Pilzes Eurotium echinulatum Delacroix, DSM 13872 im Submersverfahren, d. h. in wäßriger Aufschlämmung zu gewinnen. Das Eurotinon kann sowohl im Mycel als auch im Kulturfiltrat vorkommen, gewöhnlich befindet sich die Hauptmenge im Kulturfiltrat.

Handelt es sich um eine Flüssigkultur so wird zunächst das Mycel von der Kulturbrühe nach den üblichen Verfahren abgetrennt und anschließend wird das Eurotinon von der Zellmasse mit einem gegebenenfalls mit Wasser mischbaren organischen Lösungsmittel extrahiert. Die organische Lösungsmittelphase enthält den erfindungsgemäßen Naturstoff, sie wird gegebenenfalls im Vakuum konzentriert und wie unten beschrieben weiter aufgereinigt.

Das Kulturfiltrat wird gegebenenfalls mit dem Konzentrat des Mycel-Extraktes vereinigt und mit einem geeigneten, mit Wasser nicht mischbarem organischen Lösungsmittel, zum Beispiel mit n-Butanol, extrahiert. Die anschließend abgetrennte organische Phase wird ggf. im Vakuum konzentriert. Bevorzugt wird das Kulturfiltrat direkt chromatographisch aufgetrennt wie unten beschrieben.

Die Oberflächenkultur wird zweckmäßiger Weise erst gefriergetrocknet und anschließend mit Methanol oder 2-Propanol extrahiert, es können aber auch andere Lösungsmittel verwendet werden. Der erhaltene Extrakt wird anschliessend lyophilisiert.

Die weitere Aufreinigung des erfindungsgemäßen Eurotinons erfolgt durch Chromatographie an geeigneten Materialien, z.B. an Molekularsieben, an Normal-Phasenträger, wie z. B. Kieselgel, Aluminiumoxid, an Ionenaustauschern, bevorzugt aber an Adsorberharzen und an Umkehr-Phasen (reversed phase, RP). Mit Hilfe dieser Chromatographie wird das Eurotinon isoliert. Die Chromatographie erfolgt mit gepufferten wässrigen Lösungen oder Gemischen von wässrigen und organischen Lösungen.

Unter Gemischen von wässrigen oder organischen Lösungen versteht man alle mit Wasser mischbaren organischen Lösemittel, vorzugsweise Methanol, 2-Propanol und Acetonitril, in einer Konzentration von 10 bis 80% Lösemittel, vorzugsweise 15 bis 55 % Lösemittel oder auch alle gepufferten wässrigen Lösungen, die mit organischen Lösemitteln mischbar sind.

Unter gepufferten oder angesäuerten wäßrigen Lösungen versteht man z.B. Wasser, Phosphatpuffer, Ammoniumacetat, Citratpuffer in einer Konzentration von 1 mM bis 0,5 M sowie Ameisensäure, Essigsäure, Trifluoressigsäure oder allen handelsüblichen, dem Fachmann bekannten Säuren, vorzugsweise in einer Konzentration von 0,01 bis 3%, insbesondere 0,1%.

Chromatographiert wird mit einem Gradienten, der mit 100% wäßrigem Puffer beginnt und mit 100% Lösemittel, vorzugsweise 2-Propanol oder Acetonitril, endet. Zur Aufreinigung der erfindungsgemäßen Eurotinone wird vorzugsweise wird ein linearer Gradient von 10 bis 60% Acetonitril in gepufferter wässriger Lösung gefahren.

Das Eurotinon und abgeleitete chemische Derivate der Formel I können nach dem Fachmann bekannten Methoden in die entsprechenden physiologisch verträglichen Salzen übergeführt werden.

Unter physiologisch verträglichen Salzen von Verbindungen der Formel I und II versteht man sowohl deren organische als auch anorganische Salze, wie sie in Remington's Pharmaceutical Sciences (17. Auflage, Seite 1418 (1985)) beschrieben sind. Aufgrund der physikalischen und chemischen Stabilität und der Löslichkeit sind für saure Gruppen unter anderem Natrium-, Kalium-, Calciumund Ammoniumsalze bevorzugt; für basische Gruppen sind unter anderem Salze der Salzsäure, Schwefelsäure, Phosphorsäure oder von Carbonsäuren oder Sulfonsäuren, wie z.B. Essigsäure, Zitronensäure, Benzoesäure, Maleinsäure, Fumarsäure, Weinsäure und p-Toluolsulfonsäure bevorzugt.

Die vorliegende Erfindung umfaßt allen stereoisomeren Formen der Verbindungen der Formel I. In den Verbindungen der Formel I enthaltene Asymmetriezentren können alle unabhängig voneinander die S-Konfiguration oder die R-Konfiguration aufweisen. Zur Erfindung gehören alle möglichen Enantiomeren und Diastereomeren, ebenso Mischungen von zwei oder mehr stereoisomeren Formen, zum Beispiel Mischungen von Enantiomeren und/oder Diastereomeren, in allen Verhältnissen. Enantiomere sind also in enantiomerenreiner Form, sowohl als linksdrehende als auch als rechtsdrehende Antipoden, R- und S-Konfigurationen, in Form von Racematen und in Form von Mischungen der beiden Enantiomeren in allen Verhältnissen Gegenstand der Erfindung. Bei Vorliegen einer cis/trans-Isomerie sind sowohl die cis-Form als auch die trans-Form und Gemische dieser Formen in allen Verhältnissen Gegenstand der Erfindung.

Tests zur Ermittlung der biologischen Aktivitäten der Eurotinone:

Der Tyrosin-Kinase-Rezeptor KDR stellt das Testtarget dar. KDR spielt beim Wachstum von Endothel und bei der Angiogenese eine Schlüsselrolle und ist somit auch an der Entstehung von Tumoren beteiligt. KDR ist somit ein

wichtiges therapeutisches Zielmolekül für Krebserkrankungen und andere proliferative Erkrankungen. Im Test wird anhand der Phosphorylierung eines spezifischen Peptid-Substrates die Aktivität der KDR-Kinase gemessen. Neben der inhibierenden Aktivität auf die genannten Kinasen, werden auch weitere Kinasen, die ebenfalls an der Krebsentstehung bzw. in der Entzündungskaskade beteiligt sind, durch die Eurotinone gehemmt.

Aufgrund ihrer wertvollen pharmakologischen Eigenschaften eignen sich die erfindungsgemäßen Verbindungen zur speziellen Anwendung als Arzneimittel in der Human- und/oder Tiermedizin. Die erfindungsgemäßen Verbindungen können bei Krebserkrankungen verwendet werden, insbesondere als Chemotherapeutika. Aufgrund ihrer antioangiogenetischen Eigenschaften und der damit verbundenen Antitumor-Aktivität können sie insbesondere als Mittel gegen maligne Entartungen bei Tieren sowie beim Menschen eingesetzt werden.

Darüber hinaus ist ein Einsatz zur Prävention denkbar um erneutes Wachstum eines Tumors nach erfolgter Therapie zu verhindern. Die erfindungsgemäßen Eurotinone der Formel I können sowohl zur Monotherapie, wie auch in Kombinationstherapie mit anderen Zytostatika im Rahmen einer chemotherapeutischen Tumorbehandlung verwendet werden. Aufgrund der unterschiedlichen Angriffspunkte von Zytostatika und Angiogenese-Inhibitoren kann die Kombinationstherapie zu einer synergistischen Wirkung von KDR-Inhibitoren mit den bisher bekannten Zytostatika führen.

Die Erfindung betrifft auch pharmazeutische Zubereitungen, die ein oder mehrere der erfindungsgemäßen Eurotinone der Formel I sowie gegebenenfalls zusätzlich ein Zytostatikum als weiteren Wirkstoff enthalten. Bevorzugt ist die Verwendung in Mischung mit geeigneten Hilfsstoffen oder Trägermaterial. Als Trägermaterial können beim Menschen alle pharmakologisch verträglichen Trägermaterialien und/oder Hilfsstoffe verwendet werden.

Die Erfindung betrifft auch ein Verfahren zur Herstellung eines erfindungsgemäßen Arzneimittels, das dadurch gekennzeichnet ist, daß man mindestens eine der erfindungsgemäßen Verbindungen mit einem pharmazeutisch geeigneten und physiologisch verträglichen Träger und gegebenenfalls weiteren geeigneten Wirk, Zusatz- oder Hilfsstoffen in eine geeignete Darreichungsform bringt.

Die erfindungsgemäßen Arzneimittel werden im allgemeinen oral, lokal oder parenteral verabreicht, aber auch eine rektale Anwendung ist prinzipiell möglich. Geeignete feste oder flüssige galenische Zubereitungsformen sind beispielsweise Granulate, Pulver, Tabletten, Dragees, (Mikro-)Kapseln, Zäpfchen, Sirupe, Emulsionen, Suspensionen, Aerosole, Tropfen oder injizierbare Lösungen in Ampullenform sowie Präparate mit protrahierter Wirkstoff-Freigabe, bei deren Her-stellung üblicherweise Trägerstoffe und Zusätze und/oder Hilfsmittel wie Spreng-, Binde-, Überzugs-, Quellungs-, Gleit- oder Schmiermittel, Geschmacksstoffe, Süßungsmittel oder Lösungsvermittler Verwendung finden. Als häufig verwendete Träger- oder Hilfsstoffe seien z.B. Magnesiumcarbonat, Titandioxid, Laktose, Mannit und andere Zucker, Talkum, Milcheiweiß, Gelatine, Stärke, Vitamine, Cellulose und ihre Derivate, tierische oder pflanzliche Öle, Polyethylenglykole und Lösungsmittel, wie etwa steriles Wasser, Alkohole, Glycerin und mehrwertige Alkohole genannt.

Gegebenenfalls können die Dosierungseinheiten für die orale Verabreichung mikroverkapselt werden, um die Abgabe zu verzögern oder über einen längeren Zeitraum auszudehnen, wie beispielsweise durch Überziehen oder Einbetten des Wirkstoffs in Teilchenform in geeignete Polymere, Wachse oder dergleichen.

Vorzugsweise werden die pharmazeutischen Präparate in Dosierungseinheiten hergestellt und verabreicht, wobei jede Einheit als aktiven Bestandteil eine bestimmte Dosis einer oder mehrerer Verbindungen der erfindungsgemäßen Eurotinone der Formel I enthält. Bei festen Dosierungseinheiten wie Tabletten, Kapseln und Suppositorien kann diese Dosis bis zu etwa 500 mg, bevorzugt jedoch etwa 0,1 bis 200 mg, und bei Injektionslösungen in Ampullenform bis zu etwa 500 mg, vorzugsweise aber etwa 0,1 bis 100 mg, pro Tag betragen.

Die zu verabreichende Tagesdosis ist abhängig vom Körpergewicht, Alter, Geschlecht und Zustand des Patienten. Unter Umständen können jedoch auch höhere oder niedrigere Tagesdosen angebracht sein. Die Verabreichung der Tagesdosis kann sowohl durch Einmalgabe in Form einer einzelnen Dosierungseinheit oder aber in mehreren kleineren Dosierungseinheiten als auch durch Mehrfachgabe unterteilter Dosen in bestimmten Intervallen erfolgen.

In den sich anschließenden Beispielen wird die Erfindung weiter erläutert. Prozentangaben beziehen sich auf das Gewicht. Mischungsverhältnisse bei Flüssigkeiten beziehen sich auf das Volumen, wenn keine anderen Angaben gemacht wurden.

## Beispiele

### Beispiel 1

Herstellung einer Glycerinkultur von Eurotium echinulatum Delacroix, DSM 13872

30 ml Nährlösung (Malzextrakt 2,0%, Hefeextrakt 0,2%, Glucose 1,0% $(NH_4)_2HPO_4$ 0,05%, pH 6,0) in einem sterilen 100 ml Erlenmeyerkolben werden mit dem Stamm Eurotium echinulatum Delacroix, DSM 13872, beimpft und 6 Tage bei 25° C und 140 UpM auf einer rotierenden Schüttelmaschine inkubiert. 1,5 ml dieser Kultur werden anschließend mit 2,5 ml 80%igem Glycerin verdünnt und bei -135°C gelagert.

Beispiel 2

Herstellung einer Vorkultur im Erlenmeyerkolben von Eurotium echinulatum Delacroix, DSM 13872

100 ml Nährlösung (Malzextrakt 2,0%, Hefeextract 0,2%, Glucose 1,0% $(NH_4)_2HPO_4$ 0,05%, pH 6,0) in einem sterilen 300 ml Erlenmeyerkolben werden mit dem Stamm Eurotium echinulatum Delacroix, DSM 13872, beimpft und 7 Tage bei 25° C und 140 UpM auf einer rotierenden Schüttelmaschine inkubiert. 2 ml dieser Vorkultur werden anschließend für die Herstellung der Hauptkulturen verwendet.

Beispiel 3

Herstellung einer Hauptkultur Eurotium echinulatum Delacroix, DSM 13872 auf Festmedium Platten.

30 sterile 25 x 25 cm Platten werden mit 200 ml der folgenden Nährlösung 20 g/L Malzextrakt, 20 g/L Haferflocken, 2% Agar und pH 7.0 gegossen. Diese Platten werden mit 2 ml einer Vorkultur beimpft und bei 25° C inkubiert. Die maximale Produktion des erfindungsgemäßen Eurotinon ist nach ca. 360 Stunden erreicht.

Beispiel 4

Herstellung einer flüssigen Hauptkultur von Eurotium echinulatum Delacroix, DSM 13872.

Ein steriler 300 ml Erlenmeyerkolben mit 100 ml der folgende Nährlösung Malzextrakt 2,0%, Hefeextrakt 0,2%, Glucose 1,0% $(NH_4)_2$ $HPO_4$ 0,05%, pH 6 wird mit einer auf einem Schrägröhrchen (gleiche Nährlösung, jedoch mit 2% Agar) gewachsenen Kultur oder mit 2 ml einer Vorkultur (s. Beispiel 2) beimpft und auf einer Schüttelmaschine bei 140 UpM und 25° C inkubiert. Die maximale Produktion des erfindungsgemäßen Eurotinons ist nach ca. 360 Stunden erreicht. Zum Animpfen von 10 bis 200 L Fermentern genügt eine 96 bis 144 Std. alte Submerskultur (Animpfmenge ca. 10%) aus der gleichen Nährlösung. Die Bedingungen für diese Fermenter sind:

Temperatur 25° C
Rührergeschwindigkeit: 200 UpM
Belüftung 15 l $Min^{-1.}$
Durch wiederholte Zugabe von ethanolischer Polyollösung kann die Schaumbildung unterdrückt werden. Das Produktionsmaximum wird nach ca. 96 bis 144 Stunden erreicht.

Beispiel 5

Isolierung des Eurotinons aus einer Fermentation auf Festmedium

150 nach Beispiel 3 inkubierte Festmedium Platten wurden lyophilisiert und das Lyophilisat anschliessend mit Methanol (20-30 L) extrahiert. Der Methanolextrakt wurde am Vakuum auf 10 L reduziert und mit Wasser auf einen Methanolgehalt von 10% verdünnt. Der verdünnte Extrakt wurde anschliessend auf eine vorbereitete Glassäule (BPG 100, 4L Innenvolumen, Fa. Pharmacia Biotech) aufgetragen , die mit ca. 2 Liter MCI-Gel® CHP-20P Material (Adsorberharz der Fa. Mitsubishi Chemicals, Japan) gefüllt war. Eluiert wurde mit einem Gradienten von 100% Wasser nach 100% Isopropylalkohol. Der Säulendurchfluß (1L / 6min) wurde fraktioniert aufgefangen (je 1 L). Sämtliche Fraktionen wurden im KDR-Assay getestet und die aktiven Fraktionen (Fr. 6-14) zusammengefasst. Diese wurden am Vakuum auf ca. 10 L reduziert und diese Lösung erneut mit Wasser auf einen Gehalt von 10% der Ausgangslösung verdünnt. Die entstehende Lösung wurde nochmals auf eine Säule (s.o.) aufgetragen, die mit ca. 1,5 Liter MCI-Gel® CHP-20P Material gefüllt war. Eluiert wurde mit einem Gradienten von 100% Wasser nach 100 % Acetonitril. Der Säulendurchfluß (1L / 6min) wurde fraktioniert aufgefangen (je 1 L) und die im Test aktiven Fraktionen (Fr. 4-11) erneut zusammengefasst. Konzentrieren am Vakuum und anschliessende Lyophilisierung ergaben ca. 16 g braunes Pulver.

Beispiel 6

Reinigung des Eurotinons mittels Chromatographie

Ca. 2 g des nach Beispiel 5 gewonnen Pulvers wurden auf einer LUNA® 10 µ C18 (2) Säule (Größe: 50 mm

x 250 mm, Fa. Phenomenex, Deutschland) mit Vorsäule LUNA® 10 µ C18 (2) (Größe: 21.2 mm x 60 mm) aufgetragen und mit einem Gradienten von 10 % bis 40 % Acetonitril in 0,1% Ammoniumacetat/Wasser über 40 Minuten chromatographiert. Der Durchfluß des Elutionsmittels betrug 125 ml/min, die Fraktionsgröße 125 ml. In den Fraktionen 14 und 15 befand sich das Eurotinon. Die Lyophilisierung der genannten Fraktionen ergab ca. 320 mg angereichertes Eurotinon. Ein weiterer Aufreinigungsschritt mittels HPLC an der gleichen RP-18 Säule wie oben erfolgte mit einem Gradienten von 20 % nach 30 % in 30 Minuten. Die Eurotinonhaltigen Fraktionen wurden vereinigt, entsalzt und gefriergetrocknet. Dabei erhielt man 210 mg Eurotinon (Verbindung 1) (Reinheit > 95 %). Auch hier wurden alle Fraktionen der einzelnen Trennschritte im Biotest untersucht.

Beispiel 7

Methylierung von Eurotinon

35 mg (= 0,12 mmol) des gemäß Beispiel 6 gewonnen Eurotinons wurden in MeOH (5 ml) gelöst und mit Trimethylsilyldiazomethan im 6-fachen molaren Überschuß versetzt. Die Reaktionsmischung wurde 30 min. bei Raumtemperatur gerührt und anschließend zur Trockene eingeengt. Das erhaltene Gemisch wurde an einer LUNA® 5 µ C18 (2) Säule (Größe: 10 mm x 250 mm) chromatographisch aufgetrennt. Eluiert wurde mit einem Gradienten von 20 % nach 60 % Acetonitril in Wasser mit Zusatz von 0,1% Ammoniumacetat über 50 min mit einer Durchflussgeschwindigkeit von 6,5 ml/min. Der Säulendurchfluß wurde fraktioniert aufgefangen (je 6,5 ml Fraktionen). Die Fraktionen 19 - 22 enthielten 7,5 mg des Dimethylderivats 2,12-Dimethyleurotinon (Verbindung 2), die Fraktionen 23-25 enthielten 6 mg des Monomethylderivats 2-Methyleurotinon (Verbindung 3).

Beispiel 8

Charakterisierung des Eurotinons (Verbindung 1)

Die physikalisch-chemischen sowie spektroskopischen Eigenschaften des Eurotinons lassen sich wie folgt zusammenfassen:

Summenformel: $C_{15}H_{12}O_6$
Strukturfomel:

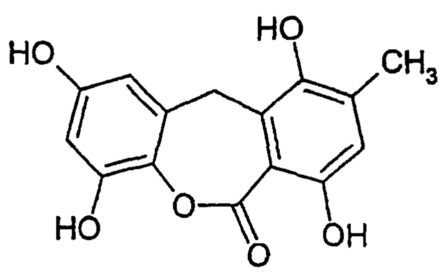

Molekulargewicht: 288
UV-Maxima: 200, 262, 330 nm
$^1H$- und $^{13}C$-NMR: siehe Tabelle 1

Tab. 1:

| NMR-chemische Verschiebungen von Eurotinon, c = 4.5 mg /ml, DMSO-D6 bei 300 K | | | | |
|---|---|---|---|---|
| **Position** | **δ ($^{13}$C)** | **m ($^{13}$C)** | **δ ($^1$H) -** | **$^nJ_{CH}$** |
| 1 | 131.45 | s | - | 6.203, 6.148 |
| 2 | 148.16 | s | - | 6.203 |
| 2-OH | - | - | 9.1 br | - |
| 3 | 101.85 | d | 6.203 | 6.145 |

Tab. 1: (fortgesetzt)

| NMR-chemische Verschiebungen von Eurotinon, c = 4.5 mg /ml, DMSO-D6 bei 300 K | | | | |
|---|---|---|---|---|
| **Position** | $\delta$ **($^{13}C$)** | **m ($^{13}C$)** | $\delta$ **($^{1}H$) -** | $^{n}J_{CH}$ |
| 4 | 154.66 | s | - | 6.203, (6.148) |
| 4-OH | - | - | 9.1 br | - |
| 5 | 104.83 | d | 6.148 | 6.203 |
| 6 | 135.13 | s br | - | - |
| 7 | 28.37 | t | 3.79 br | (6.148) |
| 8 | 132.81 | s | - | 2.147 |
| 9 | 142.49 | s | - | 6.576, 2.147 |
| 9-OH | - | - | 8.1 br | - |
| 10 | 130.34 | s | - | (6.578), (2.147) |
| 10-Me | 17.24 | q | 2.147 | 6.576 |
| 11 | 116.47 | d | 6.576 | 2.147 |
| 12 | 151.53 | s | - | 6.576 |
| 12-OH | - | - | 9.1 br | - |
| 13 | 112.19 | s | - | 6.567, (2.147) |
| 14 | 164.95 | s | - | 6.567 |

Beispiel 9

Charakterisierung des 2,12-Dimethyleurotinons (Verbindung 2)

Die physikalisch-chemischen sowie spektroskopischen Eigenschaften des 2,12-Dimethyleurotinons lassen sich wie folgt zusammenfassen:

Summenformel: $C_{17}H_{16}O_6$
Strukturformel:

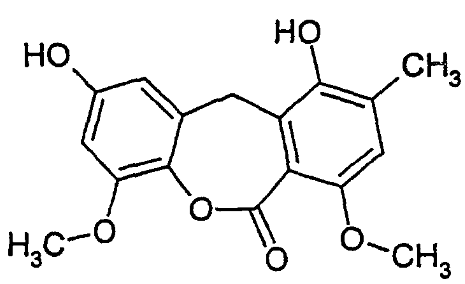

Molekulargewicht: 316
UV-Maxima: 200, 262, 330 nm
$^{1}H$- und $^{13}C$-NMR: siehe Tabelle 2

Tab. 2:

| NMR-chemische Verschiebungen des 2,12- Dimethyleurotinons, c = 2.5 mg /ml, DMSO-D6 bei 300 K | | | | | |
|---|---|---|---|---|---|
| **Position** | **δ ($^{13}$C)** | **m ($^{13}$C)** | **δ ($^{1}$H)** | **$^{n}J_{CH}$** | **NOE** |
| 1 | 131.84 | s | - | 6.311, 6.269 | - |
| 2 | 150.16 | s | - | 6.311, (6.269), 3.728 | - |
| 2-OMe | 55.47 | q | 3.728 | - | 6.311 |
| 3 | 98.57 | d | 6.311 | 9.446, 6.269, 3.728 | 9.446, 3.728 |
| 4 | 154.81 | s | - | 9.446, 6.311, 6.269 | - |
| 4-OH | - | - | 9.446 | - | 6.311, 6.269 |
| 5 | 105.56 | d | 6.269 | 9.446, 6.311 | 9.446,3.78 |
| 6 | 135.85 | s br | - | - | - |
| 7 | 28.19 | t | 3.78 br | 6.269 | 6.269, 8.385 |
| 8 | 131.35 | s | - | 8.385, (6.765), (2.205) | - |
| 9 | 143.32 | s | - | 8.385, 6.765, 2.205 | - |
| 9-OH | - | - | 8.385 | - | 3.78, 2.205 |
| 10 | 130.91 | s | - | 8.385, 6.765, 2.205 - | |
| 10-Me | 17.37 | q | 2.205 | 6.765 | 8.385, 6.765 |
| 11 | 112.95 | d | 6.765 | 3.691, 2.205 | 3.691, 2.205 |
| 12 | 151.69 | s | - | 6.765, 3.691 | - |
| 12-OMe | 56.05 | q | 3.691 | - | 6.765 |
| 13 | 114.67 | s | - | 6.765, (2.205) | - |
| 14 | 162.41 | s | - | 6.765 | - |

Beispiel 10

Charakterisierung des 2-Methyleurotinons (Verbindung 3)

Die physikalisch-chemischen sowie spektroskopischen Eigenschaften des 2- Methyleurotinons lassen sich wie folgt zusammenfassen:

Summenformel: $C_{16}H_{14}O_6$
Strukturformel:

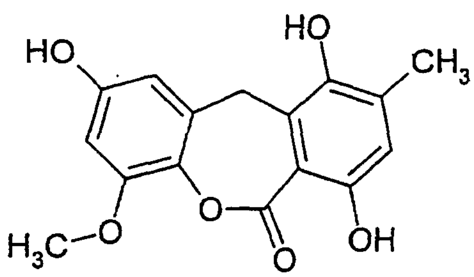

Molekulargewicht: 302
UV-Maxima: 200, 262, 330 nm

$^1H$- und $^{13}C$-NMR: siehe Tabelle 3

Tab. 3 :

| NMR-chemische Verschiebungen des 2-Methyleurotinons, c = 2.5 mg /ml, DMSO-D6 bei 300 K | | | | | |
|---|---|---|---|---|---|
| **Position** | **δ ($^{13}$C)** | **m ($^{13}$C)** | **δ ($^1$H)** | **$^nJ_{CH}$** | **NOE** |
| 1 | 132.16 | s | - | 3.864, 6.249, 6.292 | - |
| 2 | 150.21 | s | - | 3.716, 6.249 | - |
| 2- Me | 55.36 | q | 3.716 | - | 6.249 |
| 3 | 98.41 | d | 6.249 | (3.716), 6.292, 9.207 | 3.716, 9.207 |
| 4 | 155.27 | s | - | 9.207, 6.292, 6.249 - | |
| 4-OH | - | - | 9.207 | - | 6.292, 6.249 |
| 5 | 105.54 | d - | 6.292 | 9.207, 6.249, 3.864 | 9.207, 3.864 |
| 6 | 134.64 | s br | - | 3.684 | - |
| 7 | 28.19 | t | 3.864 | 6.292 | 6.292 |
| 8 | 134.62 | s | - | 7.994, 2.144, (9.344) - | |
| 9 | 142.78 | s | - | 7.994, 6.534, 2.144, (3.864) | - |
| 9-OH | - | - | 7.994 | - | 2.144 |
| 10 | 129.16 | s | - | 7.994, (6.534), (3.864), (2.144) | - |
| 10-Me | 17.34 | q | 2.144 | 6.534 | 7.994, 6.534 |
| 11 | 116.59 | d | 6.534 | 9.344,2.144 | 9.344,2.144 |
| 12 | 153.30 | s | - | 9.344, 6.534 | - |
| 12-OH | - | - | 9.344 | - | 6.534 |
| 13 | 110.38 | s | - | 9.344, 6.534, (2.144) | - |
| 14 | 166.90 | s | - | 6.534 | - |

Beispiel 11:

Untersuchung der KDR-Kinase Aktivität

Zur Bestimmung der KDR- Kinase wird mit gereinigtem Enzym in 384 well Mikrotiterplatten (Coated Flash-Plates NEN Life Science) gearbeitet. Die Enzymaktivitäten werden mittels der Phosphorylierung eines spezifischen Peptidsubstrates bestimmt. Eine vorher vorbereitete Verdünnungsreihe der Eurotinone mit Konzentrationen von 100, 50, 25, 12.5, 6.25, 3.125, 1.5625, 0.7813, 0.3906, 0.195, 0.094, 0.047 und 0 µM wird in der entsprechenden Reihenfolge in die Versuchsansätze pipettiert. Anschließend erfolgt die Zugabe des Reaktionsgemisches (radioaktiv markiertes ATP, Pufferlösung pH 7,4 und Enzymlösungen) und Inkubation für 1 h bei RT.

Beschreibung des KDR-Assays:

Material und Methoden:

Platten: 384-well FlashPlates von NEN life science.
Reader: Wallac MicroBeta Trilux counter
Reagenzien:

Substrat-Peptid: PLCγ1
Enzym: KDR-Kinase (VEGF-Rezeptor)
Kinase-Puffer:

50mM MOPS, pH7.4, 10mM $MgCl_2$, 2mM DTT, 2.5mM EDTA,
10mM β-Glycerolphosphat, 1mM Ortho-vanadat und 1mM Natriumfluorid.
Lösung zum Beschichten:

20 μg/ml Peptidsubstrat in PBS Puffer (kein $Mg^{++}$, kein $Ca^{++}$)
ATP-Lösung: 25μCi/ml $^{33}P$-γ-ATP und 12.5 μM kaltes ATP
KDR-Enzymlösung: 3.5μg/ml in Kinase-Puffer
Waschlösung : PBS (kein $Mg^{++}$, kein $Ca^{++}$)

384-well FlashPlates werden mit 60 μl (1.2 μg/well) des Peptidsubstrates bei 4°C über Nacht beschichtet und 3 x mit jeweils 80 μl PBS gewaschen. Die so beschichteten Platten können für kürzere Zeit bei 4°C und über einen längeren Zeitraum bei -20°C gelagert werden.
Reaktion: In einem Endvolumen von 50 μl enthält der Assay
10 μl verdünnte Eurotinonlösung
20 μl Enzymlösung einer Konzentration von 3.5μg/ml (70 ng/well)
20 μl ATP-Lösung (Endkonzentration von 0.5 μCi heißem und 5μM kaltem ATP/well)
Die Reaktionsmischung wird eine Stunde bei Raumtemperatur stehen gelassen. Anschließend werden die Platten dreimal mit je 75 μl Waschlösung gewaschen und die Radioaktivität in einem MicroBeta counter (Wallac) 30 Sekunden lang gemessen.

Alle Proben werden doppelt getestet in einer Endkonzentration von 1:30. Auf jeder Platte werden 16 wells verwendet, um die totale Enzymaktivität zu überprüfen (= Enzym-Kontrolle). Weitere 16 wells werden mit 4 μg/well Casein ohne Substrat beschichtet um die nicht-spezifische Inhibierung zu testen.
Die Aktivität der KDR-Kinase wird über den Einbau von radioaktivem Phosphat in das Substrat aus ATP gemessen und daraus die inhibierende Wirkung der Eurotinone ($IC_{50}$) berechnet.
Die Inhibierung wird berechnet als:

[1-(CPM Probe-CPM nicht spez.)/(CPM Enzym-Kontr.-CPM nicht spez.)] x 100 (%).

$IC_{50}$ Werte [μM]:

| | |
|---|---|
| Eurotinon (Verbindung 1): | 22 |
| 2,12-Dimethyleurotinon (Verbindung 2) | 155 |
| 2-Methyleurotinon (Verbindung 3) | 18 |

**Patentansprüche**

1. Verbindung der allgemeinen Formel I

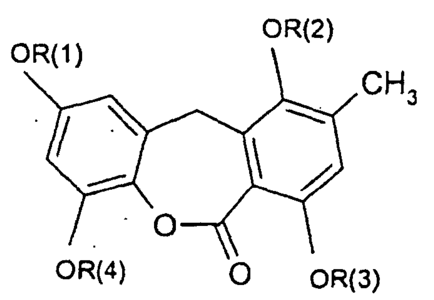

I

worin
R(1), R(2), R(3) und R(4) unabhängig voneinander Wasserstoff, ($C_1$-$C_6$)-Alkyl, ($C_2$-$C_6$)-Alkenyl, ($C_2$-$C_6$)-Alkinyl;

$C_3$-$C_6$-Cycloalkyl oder ($C_1$-$C_3$)-Alkyl-($C_3$-$C_6$)-Cycloalkyl bedeutet, wobei die obengenannten Alkylreste gegebenenfalls mit einem oder mehreren Resten substituiert sein können,
in allen ihren stereochemischen Formen und Gemische dieser Formen in jedem Verhältnis, sowie deren physiologisch verträglichen Salze.

**2.** Verbindung der Formel I gemäß Anspruch 1 worin R(1), R(2), R(3) und R(4) unabhängig voneinander Wasserstoff oder ($C_1$-$C_6$)-Alkyl bedeuten, in allen ihren stereochemischen Formen und Gemische dieser Formen in jedem Verhältnis, sowie deren physiologisch verträglichen Salze.

**3.** Verbindung der Formel I gemäß Anspruch 1 bis 2, worin R(1), R(2), R(3) und R(4) unabhängig voneinander Wasserstoff oder Methyl bedeuten, sowie deren physiologisch verträglichen Salze.

**4.** Verbindung der Formel I gemäß Anspruch 1 bis 3, worin R(1), R(2), R(3) und R(4) Wasserstoff bedeuten, sowie deren physiologisch verträglichen Salze.

**5.** Verbindung der Formel

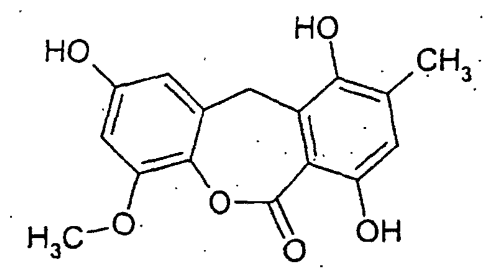

sowie deren physiologisch verträglichen Salze.

**6.** Verbindung der Formel

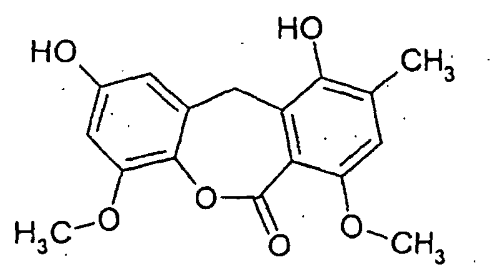

sowie deren physiologisch verträglichen Salze.

**7.** Verbindung der Formel I oder ein physiologisch verträgliches Salz davon gemäß einem oder mehreren der Ansprüche 1-6, herstellbar indem der Mikroorganismus Eurotium echinulatum Delacroix (DSM 13872) oder einer seiner Varianten oder Mutanten unter geeigneten Bedingungen in einem Kulturmedium fermentiert wird, bis sich eine oder mehrere Verbindungen der allgemeinen Formel I anhäufen und diese anschließend aus dem Kulturmedium isoliert und gegebenenfalls in ein Derivat der Formel I oder in physiologisch verträgliche Salze überführt werden.

**8.** Verfahren zur Herstellung einer Verbindung der Formel I oder ein physiologisch verträgliches Salz davon gemäß einem oder mehreren der Ansprüche 1-6, **dadurch gekennzeichnet, daß** der Mikroorganismus Eurotium echinulatum Delacroix (DSM 13872) oder einer seiner Varianten oder Mutanten unter geeigneten Bedingungen in einem Kulturmedium fermentiert wird, bis sich eine oder mehrere Verbindungen der allgemeinen Formel I in dem Kulturmedium anhäufen und anschließend aus dem Kulturmedium isoliert und gegebenenfalls in ein Derivat der Formel I oder in physiologisch verträgliche Salze überführt werden.

**9.** Verfahren gemäß Anspruch 8, **dadurch gekennzeichnet, daß** die Fermentation unter aeroben Bedingungen bei einer Temperatur zwischen 20 und 35 °C und bei einem pH zwischen 6,5 und 7,5 durchgeführt wird.

**10.** Eurotium echinulatum Delacroix (DSM 13872)

**11.** Verbindung der Formel I oder ein physiologisch verträgliches Salz davon gemäß einem oder mehreren der Ansprüche 1 - 6 zur Verwendung als Arzneimittel.

**12.** Verbindung der Formel I oder ein physiologisch verträgliches Salz davon gemäß einem oder mehreren der Ansprüche 1 - 6 zur Verwendung als Arzneimittel zur Hemmung der KDR-Kinase.

**13.** Verbindung der Formel I oder ein physiologisch verträgliches Salz davon gemäß einem oder mehreren der Ansprüche 1 - 6 zur Verwendung als Arzneimittel zur Hemmung der Angiogenese.

**14.** Pharmazeutische Zubereitung mit einem Gehalt an mindestens einer Verbindung der Formel I oder ein physiologisch verträgliches Salz davon gemäß einem oder mehreren der Ansprüche 1 - 6.

**15.** Pharmazeutische Zubereitung gemäß Anspruch 14 zusätzlich enthaltend ein Zytostatikum.

**16.** Verfahren zur Herstellung von pharmazeutischen Zubereitungen gemäß Anspruch 14, **dadurch gekennzeichnet, daß** man mindestens eine Verbindung der Formel I oder ein physiologisch verträgliches Salz davon gemäß einem oder mehreren der Ansprüche 1-6 mit geeigneten Hilfs- und/oder Trägerstoffen in eine geeignete Darreichungsform bringt.

**17.** Pharmazeutische Zubereitung gemäß Anspruch 14 und 15 zur Verwendung zur Behandlung von malignen Erkrankungen.

**18.** Verwendung von Eurotium echinulatum Delacroix (DSM 13872) zur Gewinnung von KDR-Kinase Inhibitoren.

## Claims

**1.** A compound of the formula I

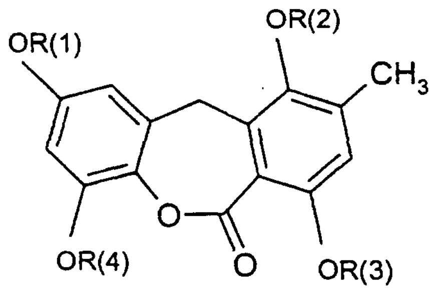

I

in which
R(1), R(2), R(3) and R(4) are, independently of each other, hydrogen, $(C_1-C_6)$-alkyl, $(C_2-C_6)$-alkenyl, $(C_2-C_6)$-alkynyl; $C_3-C_6$-cycloalkyl or $(C_1-C_3)$-alkyl-$(C_3-C_6)$-cycloalkyl, where the abovementioned alkyl radicals can, where appropriate, be substituted by one or more radicals,
in all the stereochemical forms thereof, and mixtures of these forms in any ratio, and also the physiologically tolerated salts thereof.

**2.** A compound of the formula I as claimed in claim 1, in which R(1), R(2), R(3) and R(4) are, independently of each other, hydrogen or $(C_1-C_6)$-alkyl; in all the stereochemical forms thereof, and mixtures of these forms in any ratio,

and also the physiologically tolerated salts thereof.

**3.** A compound of the formula I as claimed in claims 1 to 2, in which R(1), R(2), R(3) and R(4) are, independently of each other, hydrogen or methyl, and also the physiologically tolerated salts thereof.

**4.** A compound of the formula I as claimed in claims 1 to 3, in which R(1), R(2), R(3) and R(4) are hydrogen, and the physiologically tolerated salts thereof.

**5.** A compound of the formula

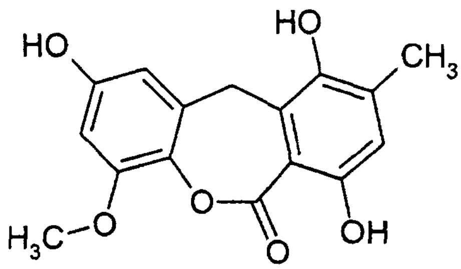

and the physiologically tolerated salts thereof.

**6.** A compound of the formula

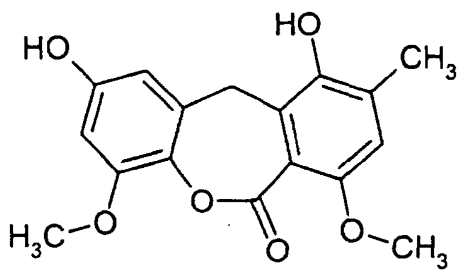

and the physiologically tolerated salts thereof.

**7.** A compound of the formula I, or a physiologically tolerated salt thereof, as claimed in one or more of claims 1- 6, which can be prepared by fermenting the microorganism Eurotium echinulatum Delacroix (DSM 13872), or one of its variants or mutants, under suitable conditions in a culture medium, until one or more compounds of the formula I accumulate, and subsequently isolating this/these compounds from the culture medium and, where appropriate, converting it/them into (a) derivative(s) of the formula I or into physiologically tolerated salts.

**8.** A process for preparing a compound of the formula I, or a physiologically tolerated salt thereof, as claimed in one or more of claims 1-6, which comprises fermenting the microorganism Eurotium echinulatum Delacroix (DSM 13872), or one of its variants or mutants, under suitable conditions in a culture medium, until one or more compounds of the formula I accumulate in the culture medium, and subsequently isolating it/them from the culture medium and, where appropriate, converting it/them into (a) derivative(s) of the formula I or into physiologically tolerated salts.

**9.** The process as claimed in claim 8, wherein the fermentation is carried out under aerobic conditions at a temperature of between 20 and 35°C and at a pH of between 6.5 and 7.5.

**10.** Eurotium echinulatum Delacroix (DSM 13872).

**11.** A compound of the formula I, or a physiologically tolerated salt thereof, as claimed in one or more of claims 1- 6, for use as a pharmaceutical.

**12.** A compound of the formula I, or a physiologically tolerated salt thereof, as claimed in one or more of claims 1- 6, for use as a pharmaceutical for inhibiting KDR kinase.

**13.** A compound of the formula I, or a physiologically tolerated salt thereof, as claimed in one or more of claims 1- 6, for use as a pharmaceutical for inhibiting angiogenesis.

**14.** A pharmaceutical preparation which has a content of at least one compound of the formula I, or a physiologically tolerated salt thereof, as claimed in one or more of claims 1- 6.

**15.** A pharmaceutical preparation as claimed in claim 14 which additionally comprises a cytostatic agent.

**16.** A process for producing pharmaceutical preparations as claimed in claim 14, which comprises bringing a compound of the formula I, or a physiologically tolerated salt thereof, as claimed in one or more of claims 1- 6, together with suitable auxiliary substances and/or carrier substances, into a suitable administration form.

**17.** A pharmaceutical preparation as claimed in claims 14 and 15 for use for treating malignant diseases.

**18.** The use of Eurotium echinulatum Delacroix (DSM 13872) for isolating KDR kinase inhibitors.

## Revendications

**1.** Composé de formule générale I

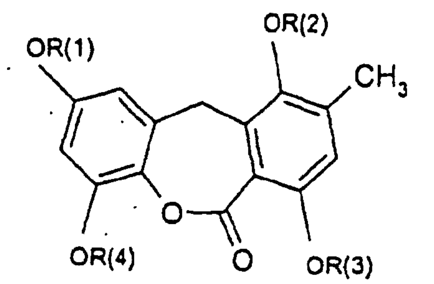

I

dans laquelle R(1), R(2), R(3) et R(4) signifient, indépendamment l'un de l'autre, hydrogène, alkyle en $C_1$ à $C_6$, alcényle en $C_2$ à $C_6$, alcynyle en $C_2$ à $C_6$, cycloalkyle en $C_3$ à $C_6$ ou (alkyle en $C_1$ à $C_3$) (cycloalkyle en $C_3$ à $C_6$), les radicaux alkyle susmentionnés pouvant le cas échéant être substitués par un ou plusieurs radicaux,
dans toutes ses formes stéréochimiques et tous les mélanges de ces formes dans tous les rapports ainsi que ses sels physiologiquement acceptables.

**2.** Composé de formule I selon la revendication 1, dans laquelle R(1), R(2), R(3) et R(4) représentent, indépendamment l'un de l'autre, hydrogène ou alkyle en $C_1$ à $C_6$, dans toutes ses formes stéréochimiques et tous les mélanges de ces formes dans tous les rapports ainsi que ses sels physiologiquement acceptables.

**3.** Composé de formule I selon la revendication 1 à 2, dans laquelle R(1), R(2), R(3) et R(4) signifient, indépendamment l'un de l'autre, hydrogène ou méthyle, ainsi que ses sels physiologiquement acceptables.

**4.** Composé de formule I selon la revendication 1 à 3, dans laquelle R(1), R(2), R(3) et R(4) signifient hydrogène, ainsi que ses sels physiologiquement acceptables.

**5.** Composé de formule

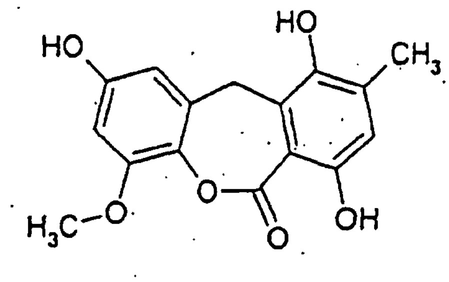

ainsi que ses sels physiologiquement acceptables.

**6.** Composé de formule

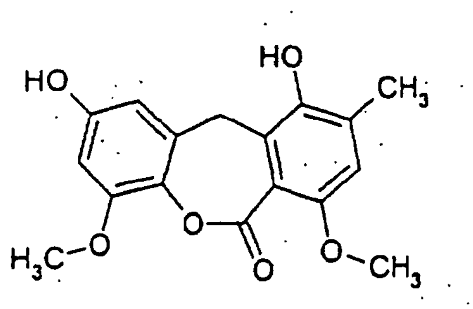

ainsi que ses sels physiologiquement acceptables.

**7.** Composé de formule I ou un sel physiologiquement acceptable de celui-ci selon l'une ou plusieurs des revendications 1 à 6, pouvant être préparé en ce que le microorganisme Eurotium echinulatum Delacroix (DSM 13872) ou une de ses variantes ou un de ses mutants est fermenté dans des conditions appropriées dans un bouillon de culture jusqu'à l'accumulation d'un ou de plusieurs composés de formule générale I et ceux-ci sont ensuite isolés du bouillon de culture et le cas échéant transformés en un dérivé de formule I ou en des sels physiologiquement acceptables.

**8.** Procédé pour la préparation d'un composé de formule I ou d'un sel physiologiquement acceptable de celui-ci selon l'une ou plusieurs des revendications 1 à 6, **caractérisé en ce qu'**on fermente le microorganisme Eurotium echinulatum Delacroix (DSM 13872) ou une de ses variantes ou un de ses mutants dans des conditions appropriées dans un bouillon de culture jusqu'à ce qu'un ou de plusieurs composés de formule générale I s'accumulent dans le bouillon de culture et ceux-ci sont ensuite isolés du bouillon de culture et le cas échéant transformés en un dérivé de formule I ou en sels physiologiquement acceptables.

**9.** Procédé selon la revendication 8, **caractérisé en ce que** la fermentation est réalisée dans des conditions aérobies à une température entre 20 et 35°C et à un pH entre 6,5 et 7,5.

**10.** Eurotium echinulatum Delacroix (DSM 13872).

**11.** Composé de formule I ou un sel physiologiquement acceptable de celui-ci selon l'une ou plusieurs des revendications 1 à 6, destiné à une utilisation comme médicament.

**12.** Composé de formule I ou un sel physiologiquement acceptable de celui-ci selon l'une ou plusieurs des revendications 1 à 6, destiné à une utilisation comme médicament en vue d'inhiber la KDR-kinase.

**13.** Composé de formule I ou un sel physiologiquement acceptable de celui-ci selon l'une ou plusieurs des revendications 1 à 6, destiné à une utilisation comme médicament en vue d'inhiber l'angiogenèse.

**14.** Préparation pharmaceutique présentant une teneur en au moins un composé de formule I ou un sel physiologiquement acceptable de celui-ci selon l'une ou plusieurs des revendications 1 à 6.

**15.** Préparation pharmaceutique selon la revendication 14 contenant en outre un cytostatique.

**16.** Procédé pour la préparation de préparations pharmaceutiques selon la revendication 14, **caractérisé en ce qu'**on amène au moins un composé de formule I ou un sel physiologiquement acceptable de celui-ci selon l'une ou plusieurs des revendications 1 à 6 avec des adjuvants et/ou des supports appropriés dans une forme d'administration appropriée.

**17.** Préparation pharmaceutique selon la revendication 14 ou 15 destinée à une utilisation pour le traitement de maladies malignes.

**18.** Utilisation de Eurotium echinulatum Delacroix (DSM 13872) pour la production d'inhibiteurs de la KDR-kinase.